# FASCICULE DE BREVET EUROPEEN

(11) **EP 0 846 008 B1**
(45) Date de publication et mention de la délivrance du brevet: **24.04.2002**
(21) Numéro de dépôt: 96929349.7
(22) Date de dépôt: 20.08.1996
(51) Int. Cl.: A61M 5/168

(54) **DISPOSITIF D'INJECTION POUVANT FONCTIONNER EN CONTINU**
VORRICHTUNG ZUR KONTINUIERLICHEN INJEKTION
CONTINUOUSLY OPERATING INFUSION DEVICE

(30) Priorité: 25.08.1995 FR 9510094
(43) Date de publication de la demande: 10.06.1998
(73) Titulaire: DEBIOTECH S.A., 1000 Lausanne 9 (CH)
(72) Inventeur: NEFTEL, Frédéric, CH-1009 Lausanne (CH); BOUVIER, Bernard, F-95610 Eragny-sur-Oise (FR)
(74) Mandataire: Dronne, Guy
(86) Numéro de dépôt international: FR9601297
(87) Numéro de publication internationale: WO9707842

(56) Documents cités:
- EP-A- 0 473 240
- EP-A- 0 650 739
- WO-A-92/11881
- DE-A- 4 333 266
- US-A- 5 328 463

## Description

L'invention concerne un dispositif et un procédé d'injection pouvant fonctionner en continu.

Pour certaines applications médicales ou certains examens médicaux, tels que la radiologie par scanner (R.X. ou R.M.N.), il est nécessaire d'effectuer une injection au patient.

Dans certains cas, cette injection doit se poursuivre en continu pour le même patient et, dans d'autres cas, le dispositif doit être rapidement opérationnel afin que le passage d'un patient à un autre patient s'effectue très rapidement, c'est-à-dire sans avoir à réinstaller tout le dispositif d'injection. On souhaite donc optimiser le temps de préparation d'une injection entre chaque patient.

De plus, on se trouve parfois en présence d'un liquide d'injection coûteux d'où le besoin d'un dispositif qui permette de limiter les pertes en liquide lors de l'injection et notamment pendant le changement du flacon contenant ce liquide.

Il serait encore plus avantageux d'utiliser tout le contenu d'un flacon d'injection avec plusieurs patients et d'injecter à chaque patient la quantité de liquide qui se révèle nécessaire pendant l'examen.

Les dispositifs d'injection utilisés jusqu'à présent ne remplissent pas les conditions énoncées précédemment voir en particulier le document EP-A-473240 ou le document EP-A-650739 : on prévoyait la quantité de produit nécessaire pour l'examen d'un patient. Dans le cas où cette quantité s'avérait insuffisante, il n'était pas possible d'en rajouter sans créer une rupture dans l'injection et, dans le cas où cette quantité était trop importante, le reste du produit était jeté du fait des risques de contamination.

Le dispositif d'injection selon l'invention a pour but de remédier aux inconvénients des dispositifs de l'état de la technique qui viennent d'être mentionnés.

Conformément à l'invention, ce but est atteint grâce au fait que le dispositif d'injection comprend deux sources d'alimentation indépendantes d'au moins un liquide destiné à être injecté à un patient par une tubulure, le dispositif d'injection comprenant, pour chacune des sources d'alimentation, des moyens d'interruption de l'écoulement du liquide et des moyens pour détecter que l'injection du liquide contenu dans la source d'alimentation est terminée, les moyens d'interruption étant commandés par les moyens de détection.

Les avantageuses dispositions suivantes sont, en outre, de préférence adoptées :
- chaque source d'alimentation est un flacon d'injection, le dispositif d'injection comprenant en outre un dispositif d'injection amont composé d'un premier tube d'alimentation, d'un deuxième tube d'alimentation et, pour chacun des flacons d'injection, de moyens de liaison, chacun des premier et deuxième tubes d'alimentation étant relié, grâce aux moyens de liaison, à un des flacons d'injection, et un dispositif d'injection aval comprenant un tube aval et des moyens de pompage du liquide, le tube aval reliant les moyens de pompage au patient;
- le dispositif d'injection amont comprend en outre des moyens de raccordement uniques solidaires des premier et deuxième tubes d'alimentation pouvant être branchés aux moyens de pompage;
- les moyens de pompage comprennent des moyens antiretour empêchant que le liquide circulant dans le dispositif d'injection aval ne remonte dans le dispositif d'injection amont; et,
- certains des moyens de liaison, d'une part, et les moyens de raccordement et les moyens de pompage d'autre part sont branchés l'un à l'autre par des moyens de connexion réalisant simultanément la fixation et l'établissement de la circulation du liquide.

Selon un autre aspect de l'invention, il est prévu un procédé qui réalise automatiquement le basculement d'une source d'alimentation à l'autre du fait que les moyens pour détecter que l'injection du liquide contenu dans la source d'alimentation est terminée commandent la fermeture des moyens d'interruption de l'écoulement du liquide de cette source d'alimentation d'une part et l'ouverture des moyens d'interruption de l'écoulement du liquide de l'autre source d'alimentation d'autre part.

L'invention sera mieux comprise, et des caractéristiques secondaires et leurs avantages apparaîtront au cours de la description du mode de réalisation donnée ci-dessous à titre d'exemple.

Il est entendu que la description et les dessins ne sont donnés qu'à titre indicatif et non limitatif.

Il sera fait référence aux dessins annexés, dans lesquels :
- la figure 1 est une vue éclatée qui représente en coupe partielle les différentes pièces à relier entre elles dans le dispositif d'injection conforme à l'invention;
- la figure 2 est une vue agrandie de certaines pièces de la figure 1;
- la figure 3 est une vue en coupe des moyens de raccordement selon la direction III-III de la figure 2; et
- la figure 4 représente schématiquement l'élément de support sur lequel les pièces de la figure 1 sont mises en place.

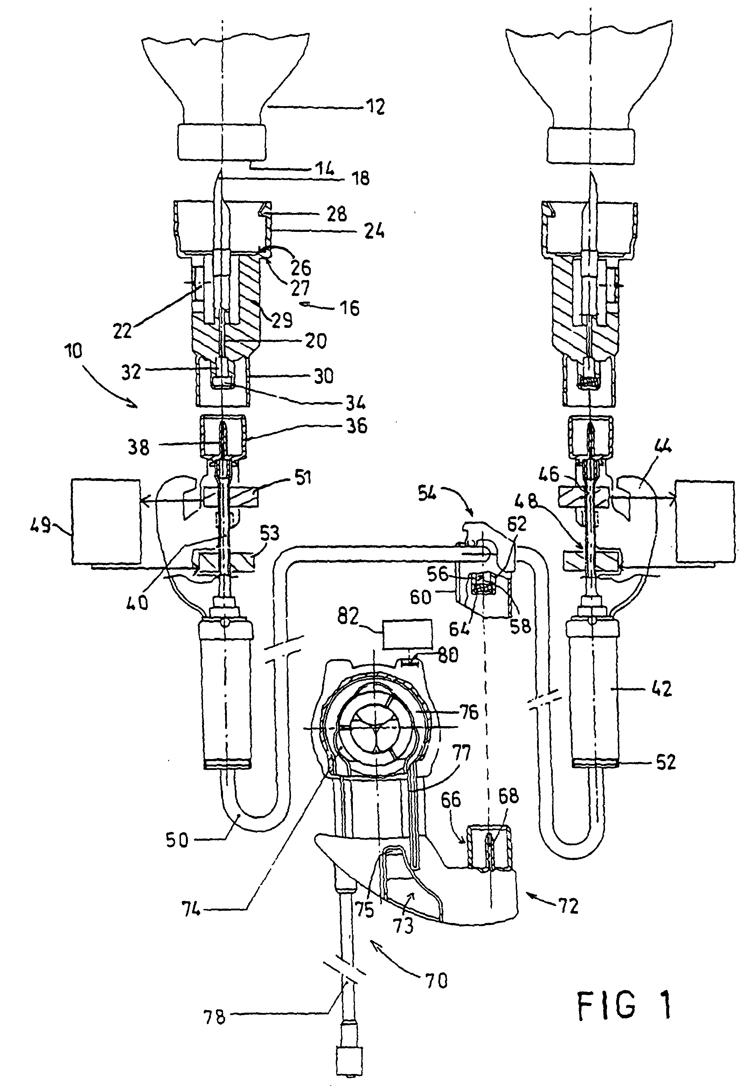

Comme on peut le voir à la figure 1, le dispositif d'injection selon l'invention est un système à double flacon.

Sur cette figure, les éléments de détection et de commande ne sont pas représentés en détail mais ils sont représentés schématiquement et leur fonctionnement sera décrit ci-après.

Le circuit d'écoulement du liquide destiné à être perfusé ou injecté au patient forme un T c'est-à-dire un double circuit amont symétrique qui converge vers un circuit aval simple branché au patient.

Le dispositif d'injection va maintenant être décrit d'amont en aval. Puisque le dispositif amont comprend deux séries d'éléments absolument identiques, nous ne décrirons que la partie gauche de ce dispositif amont tel qu'il est visible sur la figure 1 tout en sachant qu'il existe un dispositif symétrique sous forme de la partie droite du dispositif amont comprenant exactement les mêmes éléments que ceux qui vont être décrits maintenant.

Le dispositif d'injection amont 10 comprend tout d'abord un flacon 12 contenant le produit liquide destiné à être injecté au patient. Ce flacon est fermé par un opercule élastique 14 à l'extrémité avant du bouchon du flacon.

Un percuteur 16 constitue des moyens de perforation du flacon d'injection 12 car il permet de percer l'opercule 14 du flacon 12 et il est conçu de façon à permettre l'écoulement du liquide depuis le flacon 12 vers les éléments se trouvant en aval du percuteur 16. Pour cela, le percuteur 16 comprend une pointe 18 biseautée creuse prolongeant l'extrémité amont d'un conduit interne 20 qui traverse axialement le percuteur 16 de part en part. Le conduit interne 20 est relié à des moyens d'arrivée d'air sous la forme d'une prise d'air 22 permettant la pénétration de l'air dans le flacon 12 au niveau de la pointe creuse 18 afin que le flacon 12 puisse se vider de son contenu.

Il est avantageusement prévu qu'une partie du percuteur entoure la pointe 18 sous forme d'une paroi latérale 24 cylindrique de section circulaire dont le diamètre intérieur est à peine plus grand que le diamètre extérieur du bouchon du flacon. Cette paroi latérale 24 est prolongée de façon radiale vers l'intérieur par un épaulement 26 qui va servir d'élément de butée pour la face avant du bouchon du flacon. De préférence, la paroi latérale 24 porte des fentes axiales régulièrement réparties sur sa circonférence et possède un rebord annulaire 28 tourné vers l'intérieur. Ce rebord annulaire 28 forme un rebord élastique destiné à retenir l'arrière du bouchon du flacon et à éviter un mouvement de séparation axiale involontaire entre le percuteur et le flacon.

Comme on peut le voir sur la figure 1, le flacon 12 est renversé, bouchon en bas et la pointe 18 fait saillie axialement au-delà du rebord annulaire 28 de la paroi latérale 24.

Le branchement entre le flacon 12 et le percuteur 16 va maintenant être décrit. On enfonce la pointe 18 dans l'opercule 14 du flacon et on rapproche le flacon et le percuteur jusqu'à ce que le bouchon soit en butée sur l'épaulement 26. Pendant le rapprochement, la paroi latérale 24 s'est écartée grâce aux fentes mais, lorsque le bouchon est en butée, la paroi latérale reprend sa forme de départ grâce à l'élasticité engendrée par les fentes, et le rebord annulaire 28 vient en butée au-dessus de la zone latérale de la face arrière du bouchon: le percuteur 16 et le flacon 12 sont solidaires l'un de l'autre et la paroi latérale 24 permet de supporter axialement et latéralement le bouchon du flacon.

La pointe 18 permet le guidage axial entre le flacon 12 et le percuteur 16. La pointe 18, la paroi latérale 24, l'épaulement 26 et le rebord annulaire 28 constituent des moyens de support et de retenue axiale et latérale du bouchon du flacon 12 qui permettent au flacon 12 de tenir renversé verticalement sans autre moyen de fixation. La position du flacon 12 est donc entièrement assurée par le percuteur 16.

La partie médiane du percuteur 16 constitue le corps 29 du percuteur 16, ce corps de percuteur 29 est de préférence de forme extérieure générale cylindrique, de section circulaire, et se prolonge en amont par un épaulement radial externe 27 qui permet de raccorder la paroi latérale 24 au corps de percuteur 29.

La partie aval du percuteur 16 forme un tronçon cylindrique 30 de section circulaire dont l'axe coïncide avec l'axe du conduit interne 20. Le conduit interne 20 se termine en aval par une paroi cylindrique 32 bouchée à son extrémité par une membrane ou un septum en latex préfendu 34. La paroi cylindrique 32 est entourée par le tronçon cylindrique 30 qui dépasse axialement au-delà de la membrane 34.

La partie aval du percuteur 16 se raccorde à un élément rigide 36 cylindrique en forme de pot de section circulaire dont le diamètre extérieur est sensiblement égal au diamètre intérieur du tronçon cylindrique 30 afin qu'il y ait raccord par coulissement axial et engagement de coulissement télescopique avec ou sans friction entre l'élément rigide 36 et la partie aval 30 du percuteur 16. Le fond de l'élément rigide 36 est une butée pour l'extrémité aval du tronçon cylindrique 30 de la partie aval du percuteur 16. L'élément rigide 36 comprend en outre une pointe creuse 38 axiale entourée par les parois verticales de l'élément rigide 36, cette pointe 38 va pénétrer dans le septum préfendu 34 et être partiellement contenue dans la partie aval du conduit interne 20 du percuteur lorsque l'élément rigide 36 est raccordé au percuteur 16.

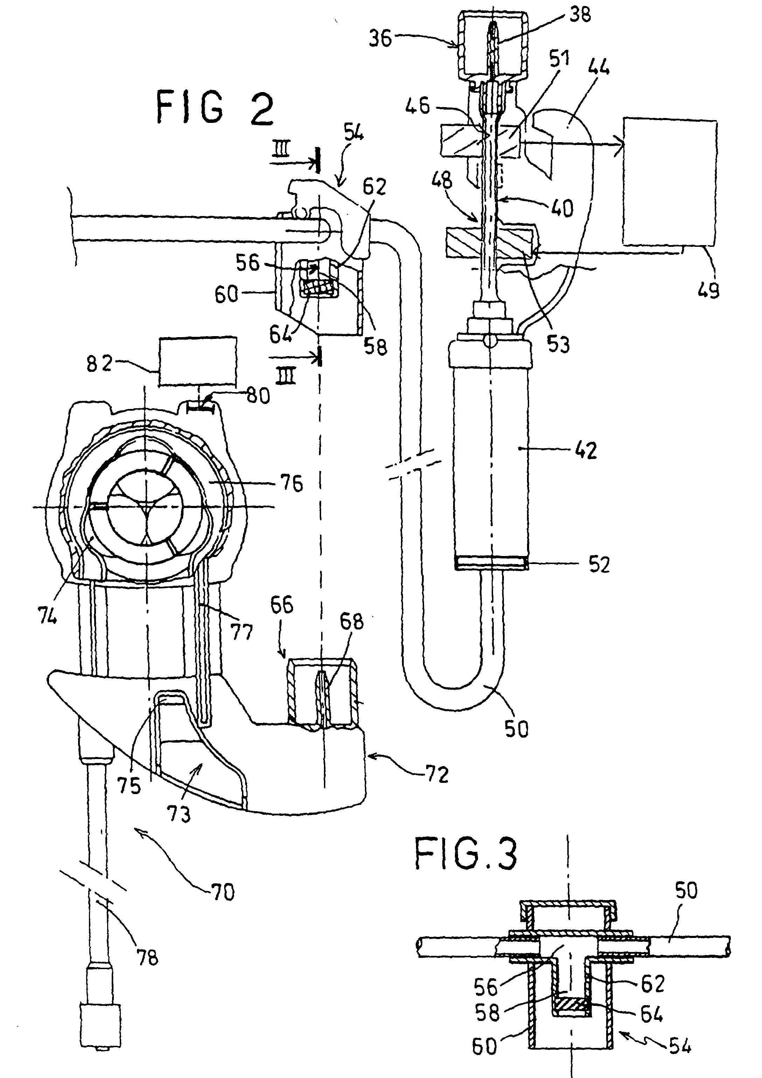

Comme il est représenté à la figure 2, un tube souple et élastique 40 est branché d'une part à l'extrémité aval de la pointe creuse 38 de l'élément rigide 36 et d'autre part à la partie amont d'une chambre à goutte 42. L'ensemble formé par l'élément rigide 36, le tube souple 40 et la chambre à goutte 42 est maintenu aligné par un support rigide en forme d'oreille 44 qui relie la partie aval de l'élément rigide 36 à la partie amont de la chambre à goutte 42 en entourant partiellement le tube souple 40 de façon à créer un tronçon amont 46 du tube souple 40 et un tronçon aval 48 du tube souple 40 de part et d'autre du tronçon de tube souple entouré par le support 44.

Le percuteur 16, l'élément rigide 36, le tube souple 40 et la chambre à goutte 42 réalisent des moyens de liaison reliant le flacon 12 à un tube d'alimentation 50.

Les moyens pour détecter que l'injection du liquide contenu dans la source d'alimentation ou flacon 12 est terminée consistent soit en des moyens pour détecter que la source d'alimentation est vide tel qu'un détecteur d'air soit en des moyens de contrôle de la quantité de liquide qui s'écoule tel qu'un débitmètre qui pourra stopper l'écoulement quand la quantité de liquide écoulé correspond à une valeur déterminée et préprogrammée.

Un détecteur d'air 51 est de préférence positionné au niveau du tronçon amont 46 du tube souple. Ce détecteur 51 peut consister en tout système permettant de détecter que le tube souple n'est pas rempli de liquide mais contient de l'air provenant d'un problème d'écoulement du liquide ou du fait que le flacon 12 est vide.

Par exemple, ce détecteur d'air 51 peut être constitué de deux capteurs piézo-électriques placés en regard l'un de l'autre de part et d'autre du tube souple 40 et en contact avec l'extérieur de ce tube. Un capteur sera émetteur d'une onde ultrasonore et l'autre capteur sera récepteur de cette onde après sa propagation au travers du tube souple 40.

Selon que le tube souple 40 est rempli de liquide ou d'air, le signal électrique émis par le récepteur piézo-électrique sera différent.

Le détecteur d'air 51 permet, via un boîtier de commande 49, de commander électriquement des moyens d'interruption 53 de l'écoulement du liquide situés au niveau du tronçon aval 48 du tube souple 40. Ces moyens d'interruption 53 peuvent obturer par pincement le tube souple 40 de façon à fermer ou ouvrir le tube souple en amont de la chambre à goutte 42. On peut utiliser par exemple un électro-clamp qui sera commandé de préférence électriquement par le détecteur d'air 51 via un relais électrique. Une commande manuelle peut également être prévue pour commander les moyens d'interruption 53 de l'écoulement du liquide: elle n'est pas prévue pour le basculement d'un flacon à l'autre, qui s'effectue automatiquement comme décrit plus loin, mais uniquement lorsque l'on change un des deux flacons ou lorsque l'on installe les deux flacons du dispositif au départ. Dans les deux cas, il est alors nécessaire de purger les tubes d'alimentation 50 et/ou les tubes souples 40 de leur air en les remplissant de liquide en commandant manuellement les moyens d'interruption 53 de l'écoulement du liquide.

La chambre à goutte 42 est un tube cylindrique transparent souple et élastique à section circulaire, de diamètre très supérieur à celui du tube souple 40, et dont les deux extrémités sont fermées par un couvercle au travers duquel débouche soit le tube souple 40, du côté amont, soit le tube d'alimentation 50, du côté aval.

L'extrémité aval de la chambre à goutte 42 porte de préférence un filtre 52 circulaire qui retient dans la chambre à goutte les particules qui pourraient avoir pénétré dans le dispositif de perfusion : par exemple des particules de caoutchouc ou des particules de plastique issues de l'opercule 14 du flacon 12, de la membrane 34 ou de la pointe biseautée 18 du percuteur 16. Ce filtre 52 sert également de brise-jet lorsque des débits importants (10 ml/s ou plus) passent dans le dispositif d'injection. On pourra utiliser un filtre en plastique avec des ouvertures de 25 µm.

Le contenu de la chambre à goutte 42 sert de stock tampon et de piège à bulles, notamment dans le cas du changement du flacon qui sera décrit plus loin. En effet, le stock de liquide que contient la chambre à goutte 42 permet d'éviter que de l'air arrive dans le tube d'alimentation 50 placé en aval de la chambre à goutte 42: si de l'air pénétrait au niveau d'un tube d'alimentation 50, il faudrait purger tout le dispositif d'injection amont 10 de son air et on perdrait du liquide.

Le premier tube d'alimentation 50 et le deuxième tube d'alimentation 50 du dispositif amont 10 se rejoignent de façon solidaire au niveau des moyens de raccordement 54 qui se présentent de préférence sous la forme d'un élément rigide avec conduit interne 56 en forme de T, visible sur la figure 3, qui permet au double circuit amont de former une seule sortie 58. Chacune des deux extrémités d'entrée du conduit interne 56 en forme de T est solidaire de l'un des premier et deuxième tubes d'alimentation 50.

L'élément rigide 54 présente en outre, dans sa partie de sortie, une structure très proche de la partie aval du percuteur 16 : un tronçon cylindrique 60 de section circulaire entoure l'extrémité de la sortie 58 du conduit interne 56 qui est une paroi cylindrique 62 bouchée à son extrémité par un septum en latex préfendu 64.

Ce septum 64 joue le même rôle que le septum 34 du percuteur 16 : son ouverture permet l'introduction d'un élément de perforation en forme de pointe qui sera maintenu et, lorsqu'on retire cette pointe, le septum se referme du fait de son élasticité ce qui garantit l'étanchéité du circuit en amont de ce septum. Le septum 64 constitue des moyens d'étanchéité qui bouchent la troisième extrémité du conduit interne 56 et qui sont destinés à coopérer avec l'extrémité amont des moyens de pompage.

La partie de sortie de l'élément rigide 54 est destinée à être raccordée à un élément rigide 66 cylindrique, avec pointe creuse 68, de structure identique à celle de l'élément rigide 36. L'élément rigide 66 est placé en amont des moyens de pompage 72 et est destiné à coopérer avec l'élément rigide 54 de la même façon que l'élément rigide 36 avec la partie aval du percuteur 16. Ainsi, comme pour le septum 34 du percuteur 16, le septum préfendu 64 est destiné à entourer de façon élastique une pointe creuse 68 située à l'extrémité amont des moyens de pompage 72. Un engagement de coulissement télescopique entre le tronçon cylindrique 60 et l'élément rigide cylindrique 66 permet de les fixer l'un à l'autre et entraîne la pénétration de la pointe creuse 68 dans le septum 64. Néanmoins, le coulissement entre les parties cylindriques des éléments rigides 66 et 54 n'est pas forcément à friction du fait que ces éléments sont supportés par ailleurs par un module moteur 84.

Les moyens de raccordement 54 comprennent en outre des moyens d'obturation partielle du conduit interne 56 permettant la circulation du liquide soit depuis les deux extrémités d'entrée du conduit interne 56 vers l'extrémité de sortie 58 du conduit interne 56, soit depuis une seule des deux extrémités d'entrée vers l'extrémité de sortie 58, l'autre extrémité d'entrée étant obturée.

Les moyens d'obturation partielle consistent, de préférence, en un robinet à trois voies: trois entrées et une sortie permettent, selon la position en rotation de ce robinet par rapport aux moyens de raccordement 54, d'utiliser ou non les deux arrivées de liquide. Ce robinet n'est pas destiné à être manipulé en cours d'injection, il sera utilisé par exemple avant une injection ne nécessitant qu'un seul flacon afin de condamner une branche du dispositif d'injection amont 10.

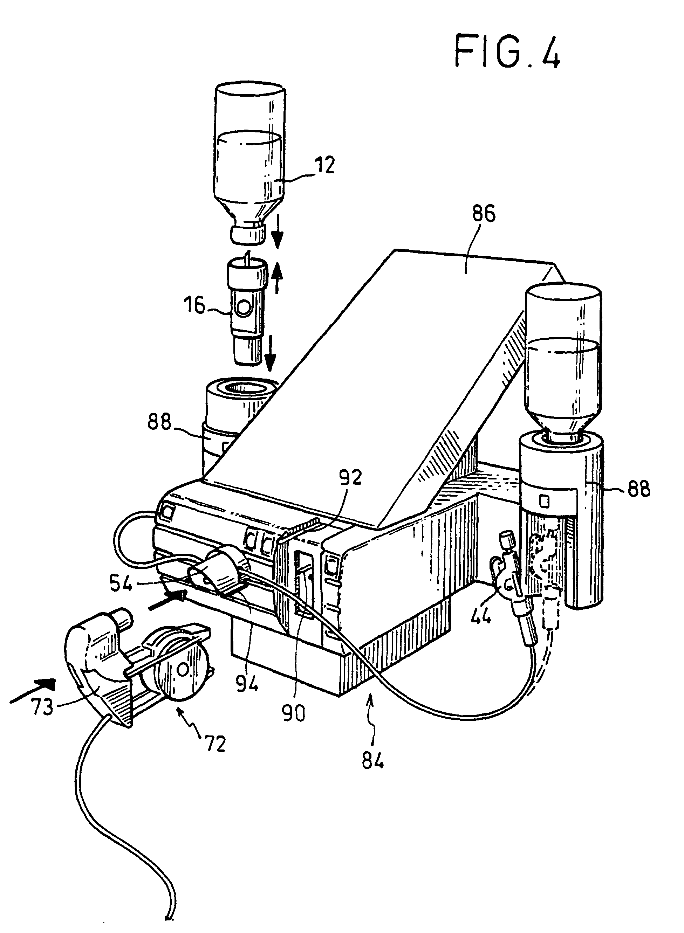

Comme représenté schématiquement sur la figure 4, le module moteur 84 constitue un corps de pompe sur lequel les autres éléments du dispositif d'injection vont être accrochés.

Le module moteur 84 comprend tout d'abord un moteur de pompe avec un arbre de sortie, un pupitre de commande 86 du dispositif d'injection et un support 88 métallique pour le percuteur 16.

Le support 88 est un cylindre de section circulaire avec un alésage axial débouchant et une fente large longitudinale qui s'étend depuis une extrémité du support 88 sur une partie de sa longueur et qui débouche sur l'alésage. L'alésage axial possède un tronçon supérieur dont le diamètre est plus large que celui du tronçon inférieur; la zone de transition entre le tronçon supérieur et le tronçon inférieur formant un épaulement annulaire. Le diamètre intérieur du tronçon inférieur correspond au diamètre extérieur du corps de percuteur 29, ce qui permet au tronçon inférieur de l'alésage du support 88 de constituer un élément annulaire qui entoure le corps de percuteur 29 avec un faible jeu d'où un mouvement transversal du percuteur très limité, son mouvement axial étant limité par butée entre l'épaulement annulaire du support 88 et l'épaulement annulaire externe 27 du percuteur 16.

Le corps de percuteur 29 et l'épaulement annulaire externe 27 du percuteur 16 constituent les zones par lesquelles le percuteur 16 est tenu. Lorsqu'il est inséré dans son support 88, le percuteur 16 est en fait complètement entouré par le support 88. Le flacon pourra être placé par l'extrémité débouchante du tronçon supérieur de l'alésage du support 88 et un système de fixation permet de positionner le support 44 en forme d'oreille dans la fente longitudinale du support 88 de sorte que la pointe 38 de l'élément rigide 36 traverse le septum 34 du percuteur 16.

Grâce au support 88, le flacon 12 reste renversé et droit malgré son poids, évitant tout mouvement du percuteur 16 qui peut être bien plus léger que le flacon si le percuteur est réalisé en plastique par exemple.

De préférence, la périphérie du corps de percuteur 29 porte un codage pour la reconnaissance du flacon 12 par le module moteur 84 des moyens de pompage avec le type et le volume du liquide contenu dans le flacon 12.

Grâce à ce codage, le dispositif d'injection selon l'invention reconnaît le flacon lors de l'insertion de l'ensemble percuteur 16 et flacon 12 dans le support 88. On peut concevoir un codage optique avec un lecteur de codes à barres par exemple ou un codage mécanique. Ce codage mécanique est, de préférence, constitué par une membrane souple qui détecte le nombre et/ou la position de pointes disposées sur la périphérie du corps de percuteur 29. Cette membrane étant disposée sur la paroi du tronçon inférieur de l'alésage du support 88.

Le module moteur 84 comprend en outre des rails 90 qui permettent le coulissement de la cassette péristaltique 72 dans le module moteur 84 de sorte que lorsqu'elle est en butée, la cassette péristaltique 72 est positionnée exactement pour que l'arbre de sortie du moteur de pompe entraîne les parties mobiles 74 de la cassette péristaltique 72 et pour que la pointe 68 de l'élément rigide 66 pénètre le septum 64. Pour que cela soit possible, l'élément rigide 54 s'accroche à un endroit du module moteur 84 prévu à cet effet qui comprend des rainures 92 de guidage. Ces rainures 92 constituent des moyens d'accrochage de l'élément rigide 54 du fait qu'elles sont complémentaires des saillies latérales de guidage 94 comprises sur l'élément rigide 54 de part et d'autre d'un axe de symétrie. Un glissement axial serré entre les saillies latérales 94 et les rainures 92 permet un guidage précis dont la position finale est atteinte par butée.

Les moyens de pompage 72, 84 se composent d'un module moteur 84 comportant des rails 90 et au moins une fente et d'une cassette péristaltique 72 comportant des rainures longitudinales 77 complémentaires des rails 90 et au moins une languette élastique 73 dont l'extrémité libre porte une patte d'accrochage 75 destinée à venir s'insérer dans ladite fente pour le positionnement précis entre le module moteur 84 et la cassette péristaltique 72.

Le corps de la cassette péristaltique 72 porte deux languettes élastiques 73 formant un clip qui coopère avec le logement du module moteur 84 dans lequel s'insère la cassette. Lorsqu'on insère la cassette péristaltique 72 dans le module moteur 84, elle est guidée par les rails 90 puis les languettes élastiques 73 se plient vers l'intérieur de la cassette du fait des pattes d'accrochage 75 qui sont en saillie extérieure par rapport au corps de la cassette. Une fois que la cassette 72 est complètement insérée dans le module moteur 84, les pattes 75 arrivent en regard des fentes du module moteur et, du fait de l'élasticité des languettes 73, ces pattes 75 s'insèrent dans les fentes ce qui bloque tout mouvement entre la cassette 72 et le module moteur 84. Pour libérer la cassette, il faut appuyer manuellement sur la partie des languettes 73 restée hors du module de façon à désengager les pattes 75 de leur fente.

L'élément rigide 66 constitue l'extrémité amont du dispositif d'injection aval 70 qui va maintenant être décrit en relation avec la figure 2.

Le dispositif d'injection aval 70 comprend des moyens de pompage composés, de préférence, d'une cassette péristaltique 72 et d'un module moteur 84 extérieur dont l'arbre de sortie entraîne en rotation les parties mobiles 74 de la cassette péristaltique 72. La cassette péristaltique 72 est traversée par un tube 76 dont l'extrémité amont est reliée à l'élément rigide 66.

Le module moteur 84 comporte en outre des moyens détecteurs de pression et d'air sous forme de capteurs. Lorsque la cassette péristaltique 72 est positionnée dans le module moteur 84, l'arbre de sortie et les moyens détecteurs peuvent se déplacer simultanément, de préférence horizontalement, de sorte que l'arbre de sortie coopère avec les parties mobiles 74 en vue de l'entraînement des parties mobiles 74 par l'arbre de sortie et que les moyens détecteurs se positionnent autour de la partie d'extrémité aval du tube 76 afin de vérifier la pression et l'absence d'air dans le liquide qui arrive vers le patient.

L'extrémité aval du tube 76 de la cassette péristaltique 72 est reliée à un tube aval 78 qui va guider le liquide jusqu'au patient.

Lors de l'utilisation du système d'injection selon l'invention, on insère les percuteurs 16 dans leur support 88, on relie les flacons 12 au percuteur 16 correspondant et les éléments rigides 36 au percuteur 16 correspondant, les supports rigides en forme d'oreille 44 étant fixés au support 88 de sorte que l'écoulement du liquide soit vertical entre chaque flacon 12 et la chambre à goutte 42 correspondante.

La cassette péristaltique 72 et les moyens de raccordement 54 sont encastrés de façon amovible dans le module moteur de sorte que les moyens de raccordement 54 puissent coopérer avec l'élément rigide 66 comme décrit précédemment et de sorte que l'arbre de sortie du module moteur puisse être positionné automatiquement pour entraîner en rotation les parties mobiles 74 de la cassette péristaltique 72.

La cassette péristaltique 72 et le module moteur peuvent être remplacés par une pompe péristaltique telle que l'une de celles qui ont été décrites dans les demandes de brevet français n° 2 383 333 et 2 644 212.

Le circuit complet est ensuite purgé de son air et rempli de liquide avant de le relier au patient et de commencer l'injection ou la perfusion en pompant le liquide de l'un des deux flacons 12, le tube souple 40 de l'autre flacon étant pincé par les moyens d'interruption 53 de l'écoulement du liquide.

Lorsqu'un flacon est vide, le détecteur d'air 51 commande les moyens d'interruption de l'écoulement 53 qui vont fermer le tube souple 40 correspondant au flacon vide et l'autre flacon prend le relais pour alimenter le dispositif grâce à l'ouverture automatique des moyens d'interruption 53 correspondants à cet autre flacon.

On peut donc changer le flacon vide en interrompant l'injection : il faut remplacer le flacon vide par un nouveau flacon et remplir la chambre à goutte correspondante en faisant un appel de liquide par une pression manuelle sur la paroi souple et élastique de la chambre à goutte 42, le tube souple 40 reliant cette chambre à goutte au nouveau flacon n'étant pas pincé par les moyens d'interruption de l'écoulement 53 du liquide alors que l'autre tube souple doit être obturé.

Ce système permet d'injecter au même patient jusqu'à deux fois le contenu d'un flacon sans interrompre l'injection : il suffit de pomper sur le deuxième flacon lorsque le premier flacon est vide.

Lorsque l'on change de patient, il n'est pas nécessaire de changer tous les éléments décrits et représentés, il suffit de changer tous les éléments du dispositif aval : cassette péristaltique 72 et tube aval 78. En effet, de préférence, La cassette péristaltique 72 empêche le retour vers le circuit amont 10 du liquide ayant pénétré dans le circuit aval 70 du fait qu'elle est occlusive : lorsque la cassette péristaltique 72 ne pompe pas le liquide d'amont en aval, sa position de repos est telle que le tube souple 76 qui la traverse est fermé par pincement. Cette propriété permet à la cassette péristaltique 72 de constituer des moyens antiretour du liquide sous la forme de moyens d'occlusion du tube 76 qui sont inclus dans la cassette péristaltique 72

Pour empêcher la réutilisation de la cassette péristaltique 72 avec plusieurs patients, celle-ci est de préférence munie de moyens de sécurité comprenant une languette plastique 80 autocassable qui se brise lors de l'introduction de la cassette péristaltique dans le module moteur.

Une fois que cette languette plastique 80 est brisée, une nouvelle utilisation de la cassette péristaltique 72 est rendue impossible par un système de détection 82 de l'absence de la languette 80.

Ce système comprend des capteurs électriques qui vont bloquer le fonctionnement du dispositif d'injection si ils détectent l'absence de la languette 80 lorsque l'on introduit la cassette péristaltique 72 dans le module moteur, dans une position intermédiaire précédant l'étape où la languette 80 est brisée.

Il faut que la cassette puisse néanmoins être utilisée une seule fois même si l'opérateur a besoin d'introduire à nouveau la cassette péristaltique 72 dans le module moteur 84 une fois que la languette 80 est brisée. Pour cela, on peut prévoir dans le système de détection 82 soit une temporisation, soit un système de contrôle qui autorise une utilisation effective unique du dispositif d'injection après rupture de la languette 80. Dans le cas d'une temporisation, on accorde un délai à l'opérateur pour débuter l'injection après la rupture de la languette 80.

Le point de départ de la temporisation est la rupture de la languette 80 et pendant la durée de cette temporisation, il est possible de débuter une injection alors qu'après la fin de la temporisation, il est impossible de réaliser une injection.

Quel que soit le moyen utilisé, le système de détection 82 empêche l'utilisation de la même cassette péristaltique 72 pour une deuxième injection.

Il est également possible que chaque source d'alimentation ou flacon 12 fournisse un liquide différent. Ceci peut permettre l'injection successive de deux produits différents au cours du même examen médical. Une application est la radiologie par scanner R.M.N. pour laquelle un liquide est un liquide actif appelé "produit contraste" et l'autre liquide est un liquide de purge neutre, par exemple une solution de glucose ou un sérum physiologique, qui permet de chasser vers le patient le liquide actif resté dans le dispositif d'injection aval 70 lorsque l'injection en liquide actif est terminée, c'est-à-dire lorsque le flacon de liquide actif est vide ou bien lorsque le débit de liquide actif écoulé a atteint une valeur prédéterminée. Dans ce type d'injection, on injecte une petite quantité de liquide actif et il est important d'injecter la plus grande partie du liquide actif qui est resté dans la tubulure en aval des moyens d'interruption 53 après qu'ils aient obturé le tube souple 40: le basculement automatique sur l'autre flacon permet au liquide de purge d'entraîner jusqu'au patient encore une partie du liquide actif qui correspond au liquide actif contenu dans le dispositif d'injection aval 70 lorsque le tube 40 du flacon de liquide actif est obturé.

## Revendications

1. Dispositif d'injection pouvant fonctionner en continu comprenant:
deux sources d'alimentation (12) indépendantes d'au moins un liquide destiné à être injecté à un patient par une tubulure,
un dispositif d'injection amont (10) comportant un premier tube d'alimentation (50), un deuxième tube d'alimentation (50) et des moyens de liaison (16,36,40,42) reliant chacun des premier et deuxième tubes d'alimentation (50) à une des sources d'alimentation (12), et
un dispositif d'injection aval (70) comprenant un tube aval (78) et des moyens de pompage du liquide (72, 84), ledit tube aval (78) reliant lesdits moyens de pompage (72, 84) au patient,
ledit dispositif d'injection amont (10) comprenant en outre des moyens de raccordement (54) uniques solidaires des premier et deuxième tubes d'alimentation (50) pouvant être branchés aux moyens de pompage (72, 84),
ledit dispositif d'injection présentant en outre, pour chacune des sources d'alimentation (12), des moyens d'interruption de l'écoulement du liquide (53) et des moyens pour détecter que l'injection du liquide contenu dans la source d'alimentation est terminée (51),
**caractérisé en ce que** chaque source d'alimentation est un flacon d'injection (12), **en ce que** le basculement d'une source d'alimentation (12) à l'autre est réalisé automatiquement du fait que lesdits moyens pour détecter que l'injection du liquide contenu dans la source d'alimentation est terminée (51) commandent la fermeture des moyens d'interruption de l'écoulement du liquide (53) de cette source d'alimentation (12), d'une part, et l'ouverture des moyens d'interruption (53) de l'autre source d'alimentation (12), d'autre part, et
**en ce que** les moyens pour détecter que l'injection du liquide contenu dans la source d'alimentation (12) est terminée sont des moyens pour détecter que la source d'alimentation est vide (51) comprenant un détecteur d'air (51).

2. Dispositif d'injection selon la revendication 1,
**caractérisé en ce que** les moyens pour détecter que l'injection du liquide contenu dans la source d'alimentation (12) est terminée comprenant des moyens de contrôle de la quantité de liquide qui s'écoule (51).

3. Dispositif d'injection selon la revendication 1,
**caractérisé en ce que** les moyens de pompage (72) comprennent des moyens antiretour empêchant que le liquide circulant dans ledit dispositif d'injection aval (70) remonte dans ledit dispositif d'injection amont (10).

4. Dispositif d'injection selon la revendication 1,
**caractérisé en ce que** les moyens de raccordement (54) comprennent un conduit interne (56) en forme de T dont chacune des deux extrémités d'entrée est solidaire de l'un des premier et deuxième tubes d'alimentation (50) et dont la troisième extrémité (58) ou extrémité de sortie est bouchée par des moyens d'étanchéité (64) destinés à coopérer avec l'extrémité amont (68) des moyens de pompage (72).

5. Dispositif d'injection selon la revendication 4,
**caractérisé en ce que** les moyens de raccordement (54) comprennent en outre des moyens d'obturation partielle du conduit interne (56) permettant la circulation du liquide soit depuis les deux extrémités d'entrée vers l'extrémité de sortie (58) soit depuis une seule des deux extrémités d'entrée vers l'extrémité de sortie (58), l'autre extrémité étant obturée.

6. Dispositif d'injection selon la revendication 3,
**caractérisé en ce que** les moyens de pompage (72) sont traversés par un tube (76) et **en ce que** les moyens antiretour consistent en des moyens d'occlusion du tube (76) qui sont inclus dans lesdits moyens de pompage (72), les moyens d'occlusion fermant ledit tube (76) lorsque lesdits moyens de pompage ne pompent pas.

7. Dispositif d'injection selon l'une quelconque des revendications 1 à 6,
**caractérisé en ce que** les moyens de liaison comprennent des moyens de perforation (16) du flacon d'injection permettant l'écoulement du liquide depuis le flacon vers la partie aval des moyens de perforation (16) et un tube souple et élastique (40).

8. Dispositif d'injection selon la revendication 7,
**caractérisé en ce que** les moyens de perforation sont un percuteur (16) avec pointe creuse biseautée (18) en amont d'un conduit interne (20) et un septum en latex (34) préfendu en aval dudit conduit interne (20).

9. Dispositif d'injection selon la revendication 8,
**caractérisé en ce que** le percuteur de perfusion (16) comprend en outre des moyens d'arrivée d'air (22).

10. Dispositif d'injection selon l'une quelconque des revendications 1 à 9,
**caractérisé en ce que** lesdits moyens de liaison comprennent en outre une chambre à goutte (42) disposée en amont du tube d'alimentation (50) et en aval desdits moyens d'interruption de l'écoulement du liquide (53), le contenu de ladite chambre à goutte (42) constituant un stock tampon.

11. Dispositif d'injection selon l'une quelconque des revendications 1 à 10,
**caractérisé en ce que** les moyens de liaison comprennent en outre une chambre à goutte (42) qui constitue un piège à bulles.

12. Dispositif d'injection selon la revendication 10 ou 11 et la revendication 7,
**caractérisé en ce que** le tube souple (40) est disposé en amont de ladite chambre à goutte (42) et qu'il est composé d'un tronçon amont (46) et d'un tronçon aval (48), lesdits moyens pour détecter que l'injection du liquide contenu dans la source d'alimentation est terminée (51) se trouvant en regard dudit tronçon amont (46) .

13. Dispositif d'injection selon la revendication 12,
**caractérisé en ce que** lesdits moyens d'interruption de l'écoulement du liquide (53) se trouvent au niveau dudit tronçon aval (48).

14. Dispositif d'injection selon les revendications 1 et 7,
**caractérisé en ce que** lesdits moyens d'interruption de l'écoulement du liquide sont des moyens de pincement (53) du tube souple (40).

15. Dispositif d'injection selon l'une quelconque des revendications 10 et 11,
**caractérisé en ce que** la chambre à goutte (42) comprend des moyens de filtration (52).

16. Dispositif d'injection selon l'une quelconque des revendications 10 et 11,
**caractérisé en ce que** la chambre à goutte (42) comprend des moyens de brise-jet (52).

17. Dispositif d'injection selon l'une quelconque des revendications 1 à 16,
**caractérisé en ce que** lesdits moyens de pompage (72, 84) comprennent en outre des moyens de sécurité (80, 82) qui empêchent la réutilisation des moyens de pompage (72, 84).

18. Dispositif d'injection selon la revendication 17,
**caractérisé en ce que** les moyens de sécurité (80, 82) comprennent un élément autocassable (80) et un système de détection (82) de l'absence de l'élément autocassable (80),

19. Dispositif d'injection selon la revendication 18,
**caractérisé en ce que** le système de détection (82) comprend une temporisation dont le point de départ est la rupture de l'élément autocassable (80) et pendant laquelle il est possible de débuter l'injection.

20. Dispositif d'injection selon la revendication 8,
**caractérisé en ce que** le percuteur (16) possède en outre des moyens de support et de retenue (18, 24, 26, 28) du bouchon du flacon (12) qui permettent au flacon (12) de tenir renversé verticalement sans autre moyen de fixation.

21. Dispositif d'injection selon la revendication 7,
**caractérisé en ce que** certains des moyens de liaison (16, 36, 40, 42) d'une part et les moyens de raccordement (54) et les moyens de pompage (72) d'autre part sont branchés l'un à l'autre par des moyens de connexion réalisant simultanément la fixation et l'établissement de la circulation du liquide.

22. Dispositif d'injection selon les revendications 7 et 21,
**caractérisé en ce que** les moyens de connexion des moyens de liaison (16, 36, 40, 42) comportent une paroi cylindrique (32) bouchée à son extrémité par un septum (34) et entourée par un tronçon cylindrique (30) en aval des moyens de perforation (16) d'une part et un élément rigide cylindrique (36) avec pointe creuse (38) axiale en amont du tube souple et élastique (40) d'autre part et que la fixation consiste en un engagement de coulissement télescopique entre le tronçon cylindrique (30) et l'élément rigide cylindrique (36), cet engagement entraînant la pénétration de la pointe creuse (38) dans le septum (34).

23. Dispositif d'injection selon les revendications 1 et 21,
**caractérisé en ce que** les moyens de connexion consistent **en ce que** les moyens de raccordement (54) comprennent en outre une paroi cylindrique (62) bouchée à son extrémité par un septum (64) et entourée par un tronçon cylindrique (60) et **en ce que** les moyens de pompage (72) comprennent en outre un élément rigide cylindrique (66) avec pointe creuse (68) et que la fixation consiste en un engagement de coulissement télescopique entre le tronçon cylindrique (60) et l'élément rigide cylindrique (66), cet engagement entraînant la pénétration de la pointe creuse (68) dans le septum (64).

24. Dispositif d'injection selon la revendication 8,
**caractérisé en ce que** le percuteur (16) possède en outre un corps de percuteur (29) cylindrique de section circulaire se prolongeant en amont par un épaulement radial externe (27) et **en ce que** le corps de percuteur (29) est retenu par un élément annulaire (88) destiné à entourer le corps de percuteur (29) avec butée contre l'épaulement externe (27).

25. Dispositif d'injection selon la revendication 4,
**caractérisé en ce que** les moyens d'étanchéité (64) sont constitués d'un septum préfendu destiné à entourer de façon élastique une point creuse (68) située à l'extrémité amont des moyens de pompage (72).

26. Dispositif d'injection selon les revendications 1 et 24,
**caractérisé en ce que** la périphérie du corps de percuteur (29) porte un codage pour la reconnaissance du flacon (12) par les moyens de pompage (72, 84) avec le type et le volume du liquide contenu dans ledit flacon (12).

27. Dispositif d'injection selon la revendication 1,
**caractérisé en ce que** les moyens de pompage (72, 84) se composent d'un module moteur (84) comportant des rails (90) et au moins une fente et d'une cassette péristaltique (72) comportant des rainures longitudinales (77) complémentaires des rails (90) et au moins une languette élastique (73) dont l'extrémité libre porte une patte d'accrochage (75) destinée à venir s'insérer dans ladite fente pour le positionnement précis entre le module moteur (84) et la cassette péristaltique (72).

28. Dispositif d'injection selon la revendication 27,
**caractérisé en ce que** le module moteur (84) comporte en outre un arbre de sortie et des moyens détecteurs de pression et d'air, que la cassette péristaltique (72) comporte en outre des parties mobiles (74) et un tube (76) et que lorsque la cassette péristaltique (72) est positionnée dans le module moteur (84), l'arbre de sortie et les moyens détecteurs peuvent se déplacer simultanément de sorte que l'arbre de sortie coopère avec les parties mobiles (74) et que les moyens détecteurs se positionnent autour de la partie d'extrémité aval du tube (76).

29. Dispositif d'injection selon l'une des revendications précédentes pouvant fonctionner en continu,
**caractérisé en ce que** chaque source d'alimentation (12) fournit un liquide différent.

30. Dispositif d'injection selon la revendication 29,
**caractérisé en ce que** l'un de ces liquide est un liquide actif, l'autre liquide étant un liquide de purge qui permet de chasser vers le patient le liquide actif resté dans le dispositif d'injection aval (70) lorsque l'injection en liquide actif est terminée.

## Claims

1. Injection apparatus capable of operating continuously, comprising:
two independent feed sources (12) of at least one liquid to be injected into a patient via a tube;
upstream injection apparatus (10) including a first feed tube (50), a second feed tube (50), and respective link means (16, 36, 40, 42) connecting each of the first and second feed tubes (50) to one of the feed sources (12); and
downstream injection apparatus (70) comprising a downstream tube (78) and pump means (72, 84) for pumping the liquid, said downstream tube (78) connecting said pump means (72, 84) to the patient;
said upstream injection apparatus (10) further comprising a single coupling means (54) secured to the first and second feed tubes (50) capable of being connected to the pump means (72, 84);
said injection apparatus further presenting, for each feed source (12), interrupter means (53) for interrupting the flow of the liquid and detection means (51) for detecting that injection of the liquid contained in the feed source has terminated;
**characterised in that** each feed source is an injection bottle (12), **in that** changeover from one feed source (12) to the other is performed automatically due to the fact that said means (51) for detecting that injection of the liquid contained in the feed source has terminated cause the interrupter means (53) for interrupting the flow of liquid from said feed source (12) to close and also cause the interrupter means (53) of the other feed source (12) to open, and
**in that** the means for detecting that injection of the liquid contained in the feed source (12) has terminated are means (51) for detecting that the feed source is empty comprising an air detector (51).

2. Injection apparatus according to claim 1,
**characterised in that** the means for detecting that injection of the liquid contained in the feed source (12) has terminated are means (51) for monitoring the quantity of liquid that has been passed.

3. Injection apparatus according to claim 1,
**characterised in that** the pump means (72) comprise anti-return means preventing the liquid flowing in said downstream injection apparatus (70) returning into said upstream injection apparatus (10).

4. Injection apparatus according to claim 1,
**characterised in that** the coupling means (54) comprise a T-shaped internal duct (56) having two inlet ends secured to respective ones of the first and second feed tubes (50) and having a third or outlet end (58) closed by sealing means (64) for co-operating with the upstream end (68) of the pump means (72).

5. Injection apparatus according to claim 4,
**characterised in that** the coupling means (54) further comprise partial closure means for partially closing the internal duct (56), enabling liquid to flow either from both inlet ends towards the outlet end (58) or else from one only of the two inlet ends towards the outlet end (58) while the other end is closed.

6. Injection apparatus according to claim 3,
**characterised in that** the pump means (72) have a tube (76) passing therethrough, and **in that** the anti-return means are constituted by occlusion means for occluding the tube (76) and included in said pump means (72), the occlusion means closing said tube (76) when said pump means are not pumping.

7. Injection apparatus according to any one of claims 1 to 6,
**characterised in that** the link means comprise perforation means (16) for perforating the injection bottle to enable liquid to flow from the bottle towards the downstream portion of the perforation means (16) and a flexible and resilient tube (40).

8. Injection apparatus according to claim 7,
**characterised in that** the perforation means are constituted by a pin (16) having a bevelled hollow tip (18) upstream from an internal duct (20) and a pre-slit latex septum (34) downstream from said internal duct (20).

9. Injection apparatus according to claim 8,
**characterised in that** the perfusion pin (16) further comprises air inlet means (22).

10. Injection apparatus according to any one of claims 1 to 9,
**characterised in that** said link means further comprise a drip chamber (42) disposed upstream from the feed tube (50) and downstream from said interrupter means (53) for interrupting the flow of the liquid, the contents of said drip chamber (42) constituting a buffer supply.

11. Injection apparatus according to any one of claims 1 to 10,
**characterised in that** the link means further comprise a drip chamber (42) which constitutes a bubble trap.

12. Injection apparatus according to claim 10 or 11 and claim 7,
**characterised in that** the flexible tube (40) is disposed upstream from said drip chamber (42) and is constituted by an upstream length (46) and a downstream length (48), said means for detecting that injection of the liquid contained in the feed source has terminated (51) being located in register with said upstream length (46).

13. Injection apparatus according to claim 12,
**characterised in that** said interrupter means (53) for interrupting the flow of liquid are located level with said downstream length (48).

14. Injection apparatus according to claims 1 and 7,
**characterised in that** said interrupter means for interrupting the flow of liquid are pinch means (53) for pinching the flexible tube (40).

15. Injection apparatus according to claim 10 or 11,
**characterised in that** the drip chamber (42) includes filter means (52).

16. Injection apparatus according to claim 10 or 11,
**characterised in that** the drip chamber (42) includes splash plug means (52).

17. Injection apparatus according to any one of claims 1 to 16,
**characterised in that** said pump means (72, 84) further comprise safety means (80, 82) which prevent the pump means (72, 84) being reused.

18. Injection apparatus according to claim 17,
**characterised in that** the safety means (80, 82) comprise an automatically breakable element (80) and a detector system (82) for detecting the absence of the automatically breakable element (80).

19. Injection apparatus according to claim 18,
**characterised in that** the detection system (82) includes a timer whose starting point is breaking of the automatically breakable element (80) and which allows injection to be started during its timer period.

20. Injection apparatus according to claim 8,
**characterised in that** the pin (16) further possesses means (18, 24, 26, 28) for supporting and retaining the cap of the bottle (12) enabling the bottle (12) to be held vertically upside-down without further fixing means.

21. Injection apparatus according to claim 7,
**characterised in that** some of the link means (16, 36, 40, 42), on the one hand, the coupling means (54) and the pump means (72), on the other hand, are interconnected by connection means that simultaneously perform fixing and establish liquid flow.

22. Injection apparatus according to claims 7 and 21,
**characterised in that** the connection means of the link means (16, 36, 40, 42) include a cylindrical wall (32) closed at its end by a septum (34) and surrounded by a cylindrical length (30) downstream from perforation means (16) and also a rigid cylindrical element (36) having an axial hollow tip (38) upstream from the flexible and resilient tube (40), and that fixing consists in telescopic sliding engagement between the cylindrical length (30) and the cylindrical rigid element (36), said engagement causing the hollow tip (38) to penetrate through the septum (34).

23. Injection apparatus according to claims 1 and 21,
**characterised in that** the connection means consist **in that** the coupling means (54) further comprise a cylindrical wall (62) closed at its end by a septum (64) and surrounded by a cylindrical length (60), and **in that** the pump means (72) further comprise a cylindrical rigid element (66) having a hollow tip (68) and **in that** fixing consists in telescopic sliding engagement between the cylindrical length (60) and the cylindrical rigid element (66), said engagement causing the hollow tip (68) to penetrate through the septum (64).

24. Injection apparatus according to claim 8,
**characterised in that** the pin (16) further possesses a cylindrical pin body (29) of circular section that is extended upstream by an outer radial shoulder (27), and **in that** the pin body (29) is retained by an annular element (88) for surrounding the pin body (29) and coming into abutment against the outer shoulder (27).

25. Injection apparatus according to claim 4,
**characterised in that** the sealing means (64) are constituted by a pre-slit septum for surrounding in elastic manner a hollow tip (68) situated at the upstream end of the pump means (72).

26. Injection apparatus according to claims 1 and 24,
**characterised in that** the periphery of the pin body (29) carries coding to enable the pump means (72, 84) to recognize the bottle (12), including the type and the volume of liquid contained in said bottle (12).

27. Injection apparatus according to claim 1,
**characterised in that** the pump means (72, 74) comprise a motor module (84) including rails (90) and at least one groove, and a peristaltic cassette (72) having longitudinal grooves (77) complementary to the rails (90) and at least one resilient tongue (73) whose free end carries a catch (75) for inserting into said groove to ensure that the peristaltic cassette (72) is accurately positioned relative to the motor module (84).

28. Injection apparatus according to claim 27,
**characterised in that** the motor module (84) further includes an outlet shaft and detector means for detecting pressure and air, **in that** the peristaltic cassette (72) further includes moving parts (74) and a tube (76), and **in that** when the peristaltic cassette (72) is in position in the motor module (84), the outlet shaft and the detector means are capable of moving simultaneously so that the outlet shaft co-operates with the moving parts (74) and the detector means take up position around the downstream end portion of the tube (76).

29. An injection apparatus method according to one of the preceding claims that is capable of operating continuously,
**characterised in that** each feed source (12) delivers a different liquid.

30. An injection apparatus method according to claim 29,
**characterised in that** one of the liquids is an active liquid, the other liquid being a purge liquid for flushing into the patient any active liquid that remains in the downstream injection apparatus (70) when injection of the active liquid has terminated.

## Patentansprüche

1. Injektionsvorrichtung zum kontinuierlichen Betrieb mit
zwei voneinander unabhängigen Versorgungsquellen (12) wenigstens einer Flüssigkeit, die dazu bestimmt ist, einem Patienten über eine Schlauchleitung injiziert zu werden,
einer aufstromseitigen Injektionsvorrichtung (10) mit einem ersten Zuleitungsschlauch (50), einem zweiten Zuleitungsschlauch (50) und Verbindungsmitteln (16, 36, 40, 42), die den ersten und den zweiten Zuleitungsschlauch (50) jeweils mit einer der Versorgungsquellen (12) verbinden, und
einer abstromseitigen Injektionsvorrichtung (70) mit einem abstromseitigen Schlauch (78) und Mitteln (72, 84) zum Pumpen der Flüssigkeit, wobei dieser abstromseitige Schlauch (78) die Pumpmittel (72, 84) mit dem Patienten verbindet,
wobei die aufstromseitige Injektionsvorrichtung (10) ferner alleinige Anschlussmittel (54) umfasst, die mit dem ersten und zweiten Zuleitungsschlauch (50) fest verbunden sind und an die Pumpmittel (72, 84) angeschlossen werden können,
wobei diese Injektionsvorrichtung ferner für jede Versorgungsquelle (12) Mittel (53) zur Unterbrechung des Flüssigkeitsabflusses und Mittel (51) aufweist, die erkennen, dass die Injektion der in der Versorgungsquelle enthaltenen Flüssigkeit beendet ist,
**dadurch gekennzeichnet, dass** jede Versorgungsquelle eine Injektionsflasche (12) ist, dass der Wechsel von einer Versorgungsquelle (12) zur anderen automatisch dadurch erfolgt, dass die Mittel (51) zum Erkennen des Injektionsendes der in der Versorgungsquelle enthaltenen Flüssigkeit einerseits das Schließen der Mittel (53) zur Unterbrechung des Flüssigkeitsabflusses dieser Versorgungsquelle (12) und andererseits das Öffnen der Unterbrechungsmittel (53) der anderen Versorgungsquelle (12) steuern, und dass die Mittel (51) zum Erkennen des Injektionsendes der in der Versorgungsquelle (12) enthaltenen Flüssigkeit Mittel sind, die erkennen, dass die Versorgungsquelle leer ist und einen Luftdetektor umfassen.

2. Injektionsvorrichtung nach Anspruch 1,
**dadurch gekennzeichnet, dass** die Mittel zum Erkennen des Injektionsendes der in der Versorgungsquelle (12) enthaltenen Flüssigkeit Mittel (51) zur Kontrolle der abfließenden Flüssigkeitsmenge umfassen.

3. Injektionsvorrichtung nach Anspruch 1,
**dadurch gekennzeichnet, dass** die Pumpmittel (72) Rücklaufsperrmittel umfassen, die verhindern, dass die in der abstromseitigen Injektionsvorrichtung (70) zirkulierende Flüssigkeit in die aufstromseitige Injektionsvorrichtung gelangt.

4. Injektionsvorrichtung nach Anspruch 1,
**dadurch gekennzeichnet, dass** die Anschlussmittel (54) einen T-förmigen Innenkanal (56) umfassen, dessen beide Zugangsenden mit dem ersten bzw. zweiten Zuleitungsschlauch (50) fest verbunden sind und dessen drittes Ende (58) oder Ausgangsende durch Dichtungsmittel (64) verschlossen ist, die dazu bestimmt sind, mit dem aufstromseitigen Ende (68) der Pumpmittel (72) zusammenzuwirken.

5. Injektionsvorrichtung nach Anspruch 4,
**dadurch gekennzeichnet, dass** die Anschlussmittel (54) ferner Mittel zum partiellen Verschluss des Innenkanals (56) umfassen, die die Zirkulation der Flüssigkeit entweder von beiden Zugangsenden zum Ausgangsende (58) oder von einem der beiden Zugangsenden zum Ausgangsende (58) gestatten, wobei das andere Ende verschlossen ist.

6. Injektionsvorrichtung nach Anspruch 3,
**dadurch gekennzeichnet, dass** die Pumpmittel (72) von einem Schlauch (76) durchquert werden und dass die Rücklaufsperrmittel aus Mitteln zum Verschließen des Schlauches (76) bestehen, die in den Pumpmitteln (72) beinhaltet sind, wobei die Verschlussmittel den Schlauch (76) schließen, wenn die Pumpmittel nicht pumpen.

7. Injektionsvorrichtung nach einem der Ansprüche 1 bis 6,
**dadurch gekennzeichnet, dass** die Verbindungsmittel Mittel (16) zur Perforierung der-Injektionsflasche umfassen, die das Abfließen der Flüssigkeit aus der Flasche zu dem abstromseitigen Teil der Perforierungsmittel und einem flexiblen und elastischen Schlauch (40) gestatten.

8. Injektionsvorrichtung nach Anspruch 7,
**dadurch gekennzeichnet, dass** die Perforierungsmittel aus einem Perforator (16) mit einer abgeschrägten hohlen Spitze (18) aufstromseitig eines Innenkanals (20) und einer geschlitzten Scheidewand (34) aus Latex abstromseitig dieses Innenkanals (20) bestehen.

9. Injektionsvorrichtung nach Anspruch 8,
**dadurch gekennzeichnet, dass** der Infusionsperforator (16) ferner Mittel (22) zur Luftzufuhr umfasst.

10. Injektionsvorrichtung nach einem der Ansprüche 1 bis 9,
**dadurch gekennzeichnet, dass** die Verbindungsmittel ferner eine Tropfkammer (42) umfassen, die aufstromseitig des Zuleitungsschlauchs (50) und abstromseitig der Mittel (53) zur Unterbrechung des Flüssigkeitsabflusses angeordnet sind, wobei der Inhalt dieser Tropfkammer (42) einen Puffervorrat bildet.

11. Injektionsvorrichtung nach einem der Ansprüche 1 bis 10,
**dadurch gekennzeichnet, dass** die Verbindungsmittel ferner eine Tropfkammer (42) umfassen, die einen Blasenfänger bildet.

12. Injektionsvorrichtung nach Anspruch 10 oder 11 und Anspruch 7,
**dadurch gekennzeichnet, dass** der flexible Schlauch (40) aufstromseitig der Tropfkammer (42) angeordnet ist und aus einem aufstromseitigen Teilstück (46) und einem abstromseitigen Teilstück (48) besteht, wobei sich die Mittel (51) zum Erkennen des Injektionsendes der in der Versorgungsquelle enthaltenen Flüssigkeit gegenüber dem aufstromseitigen Teilstück (46) befinden.

13. Injektionsvorrichtung nach Anspruch 12,
**dadurch gekennzeichnet, dass** sich die Mittel (53) zur Unterbrechung des Flüssigkeitsabflusses auf der Höhe des abstromseitigen Teilstückes (48) befinden.

14. Injektionsvorrichtung nach Anspruch 1 und 7,
**dadurch gekennzeichnet, dass** die Mittel zur Unterbrechung des Flüssigkeitsabflusses Mittel (53) zum Abklemmen des flexiblen Schlauchs (40) sind.

15. Injektionsvorrichtung nach einem der Ansprüche 10 oder 11,
**dadurch gekennzeichnet, dass** die Tropfkammer (42) Filtriermittel (52) umfasst.

16. Injektionsvorrichtung nach einem der Ansprüche 10 oder 11,
**dadurch gekennzeichnet, dass** die Tropfkammer (42) Mittel (52) zur Strahlregulierung umfasst.

17. Injektionsvorrichtung nach einem der Ansprüche 1 bis 16,
**dadurch gekennzeichnet, dass** die Pumpmittel (72, 84) ferner Sicherheitsmittel (80, 82) umfassen, die eine erneute Verwendung der Pumpmittel (72, 84) verhindern.

18. Injektionsvorrichtung nach Anspruch 17,
**dadurch gekennzeichnet, dass** die Sicherheitsmittel (80, 82) ein selbstbrechendes Element (80) und ein System (82) umfassen, welches das Fehlen des selbstbrechenden Elements (80) erkennt.

19. Injektionsvorrichtung nach Anspruch 18,
**dadurch gekennzeichnet, dass** das Erkennungssystem (82) eine Verzögerungszeit umfasst, die mit dem Bruch des selbstbrechenden Elements (80) beginnt und innerhalb derer mit der Injektion begonnen werden kann.

20. Injektionsvorrichtung nach Anspruch 8,
**dadurch gekennzeichnet, dass** der Perforator (16) ferner Trageund Haltemittel (18, 24, 26, 28) für den Flaschenstopfen (12) umfaßt, die es der Flasche (12) ermöglichen, ohne jedes weitere Befestigungsmittel kopfüber senkrecht zu stehen.

21. lnjektionsvorrichtung nach Anspruch 7,
**dadurch gekennzeichnet, dass** einerseits einige der Verbindungsmittel (16, 36, 40, 42) und andererseits die Anschlussmittel (54) und die Pumpmittel (72) durch Verknüpfungsmittel aneinander angeschlossen sind, wobei gleichzeitig die Befestigung und das Herstellen der Zirkulation der Flüssigkeit bewirkt werden.

22. Injektionsvorrichtung nach Anspruch 7 und 21,
**dadurch gekennzeichnet, dass** die Verknüpfungsmittel der Verbindungsmittel (16, 36, 40, 42) eine zylindrische Wand (32) umfassen, die an ihrem Ende durch eine Scheidewand (34) verschlossen und einerseits von einem zylindrischen Teilstück (30) abstromseitig der Perforierungsmittel (16) und andererseits von einem starren zylindrischen Element (36) mit axial hohler Spitze (38) aufstromseitig des flexiblen und elastischen Schlauchs (40) umgeben ist, und dass die Befestigung durch ein teleskopartiges Einstecken zwischen das zylindrische Teilstück (30) und das starre zylindrische Element (36) erfolgt, wobei durch dieses Einstecken die hohle Spitze (38) in die Scheidewand (34) eindringt.

23. Injektionsvorrichtung nach Anspruch 1 und 21,
**dadurch gekennzeichnet, dass** die Verknüpfungsmittel darin bestehen, dass die Anschlussmittel (54) ferner eine zylindrische Wand (62) umfassen, die an ihrem Ende durch eine Scheidewand (64) versperrt und von einem zylindrischen Teilstück (60) umgeben ist, und dass die Pumpmittel (72) ferner ein starres zylindrisches Element (66) mit einer hohlen Spitze (68) umfassen, und dass die Befestigung durch ein teleskopartiges Einstecken zwischen das zylindrische Teilstück (60) und das starre zylindrische Element (66) erfolgt, wobei durch dieses Einstecken die hohle Spitze (68) in die Scheidewand (64) eindringt.

24. Injektionsvorrichtung nach Anspruch 8,
**dadurch gekennzeichnet, dass** der Perforator (16) ferner einen zylindrischen Perforatorschaft (29) mit rundem Querschnitt besitzt, der sich aufstromseitig durch einen äußeren radialen Vorsprung (27) verlängert, und dass der Perforatorschaft (29) von einem ringförmigen Element (88) gehalten wird, das dazu bestimmt ist, den Perforatorschaft (29) gegen den äußeren Vorsprung (27) liegend zu umgeben.

25. Injektionsvorrichtung nach Anspruch 4,
**dadurch gekennzeichnet, dass** die Dichtungsmittel (64) aus einer geschlitzten Scheidewand bestehen, die dazu bestimmt ist, eine hohle Spitze (68) elastisch zu umgeben, die sich am aufstromseitigen Ende der Pumpmittel (72) befindet.

26. Injektionsvorrichtung nach Anspruch 1 und 24,
**dadurch gekennzeichnet, dass** der Umfang des Perforatorschaftes (29) mit einer Codierung hinsichtlich Art und Menge der in dieser Flasche enthaltenen Flüssigkeit zur Erkennung der Flasche (12) durch die Pumpmittel (72, 84) versehen ist.

27. Injektionsvorrichtung nach Anspruch 1,
**dadurch gekennzeichnet, dass** die Pumpmittel (72, 84) sich zusammensetzen aus einem Antriebsmodul (84) mit Schienen (90) und wenigstens einem Schlitz sowie aus einem Peristaltik-Kästchen (72) mit zu den Schienen (90) komplementären Längsrillen (77) und wenigstens einer elastischen Lasche (73), deren freies Ende eine Klammer (75) trägt, die dazu bestimmt ist, sich zur exakten Positionierung zwischen dem Antriebsmodul (84) und dem Peristaltik-Kästchen (72) in den Schlitz einzuhaken.

28. Injektionsvorrichtung nach Anspruch 27,
**dadurch gekennzeichnet, dass** das Antriebsmodul (84) zudem eine Abtriebswelle und Mittel zum Erfassen von Druck und Luft umfasst, dass das Peristaltik-Kästchen (72) ferner bewegliche Teile (74) und einen Schlauch (76) umfasst, und dass sich die Abtriebswelle und die Erfassungsmittel bei Positionierung des Peristaltik-Kästchens (72) im Antriebsmodul (84) gleichzeitig fortbewegen können, so dass die Abtriebswelle mit den beweglichen. Teilen (74) zusammenwirkt und sich die Erfassungsmittel um das abstromseitige Ende des Schlauchs (76) positionieren.

29. Injektionsvorrichtung nach einem der vorhergehenden Ansprüche zum kontinuierlichen Betrieb,
**dadurch gekennzeichnet, dass** jede Versorgungsquelle (12) eine unterschiedliche Flüssigkeit liefert.

30. Injektionsvorrichtung nach Anspruch 29,
**dadurch gekennzeichnet, dass** eine der Flüssigkeiten eine aktive Flüssigkeit und die andere eine Spülflüssigkeit ist, die es erlaubt, die in der abstromseitige Injektionsvorrichtung (70) verbliebene aktive Flüssigkeit zum Patienten zu leiten, wenn die Injektion mit aktiver Flüssigkeit beendet ist.
